# EUROPEAN PATENT SPECIFICATION

(11) **EP 2 400 298 B1**
(45) Date of publication and mention of the grant of the patent: **14.08.2013**
(21) Application number: 10005602.7
(22) Date of filing: 28.05.2010
(51) Int. Cl.: G01N 33/50

(54) **Single B-cell cultivation method and specific antibody production**
Verfahren zur Kultivierung einer einzigen B-Zelle und spezifische Antikörperproduktion
Procédé de culture de lymphocyte simple et production d'anticorps spécifque

(43) Date of publication of application: 28.12.2011
(73) Proprietor: F.Hoffmann-La Roche AG, 4070 Basel (CH)
(72) Inventor: ENDL, Josef, 82362 Weilheim (DE); HERR, Natalie, 68794 Oberhausen/Rheinhausen (DE); OFFNER, Sonja, 82377 Penzberg (DE); PLATZER, Josef, 82538 Geretsried (DE); SIEWE, Basile, Chicago IL 60657 (US); THOREY, Irmgard, 82362 Weilheim (DE)
(74) Representative: Burger, Alexander

(56) References cited:
- WO-A1-91/16418
- WO-A2-2007/031550
- US-A1- 2007 269 868
- MASRI ET AL: "Cloning and expression in E. coli of a functional Fab fragment obtained from single human lymphocyte against anthrax toxin" MOLECULAR IMMUNOLOGY, PERGAMON, GB LNKD- DOI:10.1016/J.MOLIMM.2006.09.007, vol. 44, no. 8, 1 December 2006 (2006-12-01), pages 2101-2106, XP005792748 ISSN: 0161-5890
- PIKE B L ET AL: "INTERLEUKIN 1 CAN ACT AS A B CELL GROWTH AND DIFFERENTIATION FACTOR" PROCEEDINGS OF THE NATIONAL ACADEMY OF SCIENCES OF THE UNITED STATES (PNAS), NATIONAL ACADEMY OF SCIENCE, US, vol. 82, no. 23, 1 January 1985 (1985-01-01), pages 8153-8157, XP009137385 ISSN: 0027-8424

## Description

Herein is reported a method for obtaining the amino acid sequence of at least the variable domains of a monoclonal antibody secreted by a single B-cell that has been obtained from a population of B-cells from an experimental animal by single cell deposition and co-cultivation with feeder cells in the presence of a feeder mix.

### Background of the Invention

For obtaining cells secreting monoclonal antibodies the hybridoma technology developed by Koehler and Milstein is widely used. But in the hybridoma technology only a fraction of the B-cells obtained from an immunized experimental animal can be fused and propagated. Thus, a big fraction of antibody diversity is lost. The source of the B-cells is generally an organ of an immunized experimental animal such as the spleen.

Zubler et al. started in 1984 to develop a different approach for obtaining cells secreting monoclonal antibodies. Therein the B-cells are obtained from the blood of the immunized experimental animal and co-cultivated with murine EL-4 B5 feeder cells in the presence of a cytokine comprising feeder mix. With this methodology up to 50 ng/ml antibody can be obtained after 10-12 days of co-cultivation.

Weitkamp, J-H., et al., (J. Immunol. Meth. 275 (2003) 223-237) report the generation of recombinant human monoclonal antibodies to rotavirus from single antigen-specific B-cells selected with fluorescent virus-like particles. A method of producing a plurality of isolated antibodies to a plurality of cognate antigens is reported in US 2006/0051348. In WO 2008/144763 and WO 2008/045140 antibodies to IL-6 and uses thereof and a culture method for obtaining a clonal population of antigen-specific B cells are reported, respectively.

In US 2007/269868 a culture method for obtaining a clonal population of antigen-specific B-cells is reported. The cloning and expression in E. coli of a functional Fab fragment obtained from single human lymphocyte against anthrax toxin is reported by Masri, S.A., et al. (Mol. Immunol. 44 (2007) 2101-2106). In WO 2007/031550 a method for preparing immunoglobulin libraries is reported. Tung, J.W., et al. report phenotypically distinct B cell development pathways map to the three B cell lineages in the mouse (Proc. Natl. Acad. Sci. USA 103 (2006) 6293-6298).

### Summary of the Invention

Herein is reported a method for the isolation of a B-cell from a population of B-cells, wherein within four weeks after the first immunization of the experimental animal the induced antibody producing cells can be isolated and the binding specificity of the antibodies can be determined.

One aspect as reported herein is a method for obtaining a B-cell clone comprising the following steps:
a) obtaining B-cells from the blood of a rabbit,
b) labeling IgG⁺-B-cells and/or CD 138⁺-B-cells,
c) incubating the B-cells at 37 °C for one hour in co-cultivation medium prior to depositing the labeled B-cells as single cells,
d) individually co-cultivating the single cell deposited B-cells with feeder cells,
e) selecting a B-cell clone proliferating and secreting antibody in step d) and thereby obtaining a B-cell clone.

In a further embodiment the method comprises the step of centrifuging the single cell deposited B-cells prior to the co-cultivation. In still another embodiment the method comprises after step a) and prior to step b) the following step: ab) panning the B-cells with immobilized antigen. In also an embodiment the obtaining is by a density gradient centrifugation.

In one embodiment the co-cultivation medium is an RPMI 1640 medium supplemented with 10 % (v/v) FCS, 1 % (w/v) of a 200 mM glutamine solution comprising penicillin and streptomycin, 2 % (v/v) 100 mM sodium pyruvate, 1 % (v/v) 1 M 2-(4-(2-hydroxyethyl)-1-piperazine)-ethane sulfonic acid (HEPES) buffer.

In one embodiment the labeling is of IgG⁺CD19⁺-B-cells, IgG⁺CD38⁺-B-cells, IgG⁺CD268⁺-B-cells, IgG-CD138⁺-B-cells, CD27⁺CD138⁺-B-cells or CD3-CD27⁺-B-cells. In another embodiment the B-cells are obtained from mouse and the labeling is of IgG⁺CD19⁺-B-cells and/or IgG-CD138⁺-B-cells. In a further embodiment the B-cells are obtained from hamster and the labeling is of IgG⁺IgM-B-cells. In still another embodiment the B-cells are obtained from rabbit and the labeling is of CD138⁺IgG⁺-B-cells and/or IgG⁺IgM⁻-B-cells.

In one embodiment the feeder cell is a murine EL-4 B5 cell.

In one embodiment the co-cultivating is in the presence of a feeder mix. In a further embodiment the feeder mix is a thymocyte cultivation supernatant. In another embodiment the feeder mix comprises interleukin-Ibeta and tumor necrosis factor alpha. In also an embodiment the feeder mix comprises interleukin-2 and/or interleukin-10. In one embodiment the feeder mix comprises Staphylococcus aureus strain Cowans cells. In also an embodiment the feeder mix comprises interleukin-21. In another embodiment the feeder mix comprises B-cell activation factor of the tumor necrosis factor family (BAFF). In a further embodiment the feeder mix comprises interleukin-6. In still a further embodiment the feeder mix comprises interleukin-4.

In one embodiment the method comprises the additional step of
f) determining the amino acid sequence of the variable light and heavy chain domain of the antibody produced by the selected B-cell clone of step e) by a reverse transcriptase PCR and nucleotide sequencing, and thereby obtaining a monoclonal antibody amino acid variable domain sequence.

In another embodiment the obtaining the B-cells is from an experimental animal of from 4 days to 9 days after immunization. In also an embodiment the labeling of the B-cells results in labeling of 0.1 % to 2.5 % of the cells of the total B-cell population.

Also an aspect as reported herein is a method for producing an antibody comprising the following steps
a) providing a population of mature B-cells obtained from the blood of a rabbit,
b) labeling IgG⁺-B-cells and/or CD138⁺-B-cells of the population of B-cells with at least one fluorescence dye (in one embodiment with one to three, or two to three fluorescence dyes),
c) incubating the B-cells at 37 °C for one hour in co-cultivation medium prior to depositing single cells of the labeled population of B-cells in individual containers (in one embodiment is the container a well of a multi well plate),
d) cultivating the deposited individual B-cells in the presence of feeder cells and a feeder mix (in one embodiment the feeder cells are EL-4 B5 cells, in one embodiment the feeder mix is natural TSN, in one embodiment the feeder mix is a synthetic feeder mix),
e) determining the binding specificity of the antibodies secreted in the cultivation medium of the individual B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody variable light and heavy chain variable domain encoding nucleic acid in an expression cassette for the expression of an antibody,
h) introducing the nucleic acid in a cell,
i) cultivating the cell and recovering the antibody from the cell or the cell culture supernatant and thereby producing an antibody.

### Detailed Descrintion of the Invention

The method reported herein allows for a rapid characterization of the binding specificity of individual monoclonal antibodies secreted by a population of B-cells, i.e. within four weeks after the first immunization of the experimental animal the induced antibody producing cells can be isolated and the binding specificity of the antibodies produced therefrom can be determined, i.e. at least 4 different experiments can be performed due to the antibody amount/concentration in the B-cell supernatant.

### Immunization:

Often mice are used as animal model for evaluating antibody based therapies. Therefore, it is often required to provide cross-reactive antibodies binding to the murine antigen as well as to the human antigen. The method as reported herein can be used to provide cross-reactive antibodies. In the method as reported herein B-cells obtained from mouse, hamster and rabbit can be used. In one embodiment the rabbit is selected from New Zealand White (NZW) rabbits, Zimmermann-rabbits (ZIKA), Alicia-mutant strain, basilea mutant strain, transgenic rabbits with a human immunoglobulin locus, rbIgM knock-out rabbits and mixtures thereof.

In one embodiment the rabbits chosen for immunization are not older than 12 weeks.

### Source and isolation of B-cells:

The blood of an experimental animal provides a high diversity of antibody producing B-cells. The therefrom obtained antibodies have almost no identical or overlapping amino acid sequences, thus, show a high diversity.

In one embodiment the B-cells from the blood of an experimental animal are obtained of from 4 days after immunization until 9 days after immunization. This time span allows for a high flexibility in the method as reported herein and in this time span the B-cells providing fro the most affine antibodies migrate from spleen to blood.

B-cells from the blood of an experimental animal may be obtained with any method known to a person skilled in the art. For example, density gradient centrifugation and red blood cell lysis can be used. Density gradient centrifugation compared to hypotonic lysis provides for a higher overall yield, i.e. number, of cells. Additionally from the cells obtained by density gradient centrifugation a larger number of cells divides and grows in the co-cultivation step and also the concentration of secreted antibody is higher compared to cells obtained with a different method. Therefore, in one embodiment the providing of a population of B-cells is by density gradient centrifugation.

**Table 1: Number of IgG producing wells when the cells are obtained by density gradient centrifugation (DGC) or hypotonic lysis of erythrocytes.**

| | **mouse, DGC** | **mouse, lysis** | **hamster, DGC** | **hamster, lysis** |
|---|---|---|---|---|
| Number of isolated cells [x10⁶] | 1.7±0.2 (n= 2) | 1.6±0.1 (n= 2) | 2.1 ± 0.2 (n= 2) | 0.9 ± 0.1 (n= 2) |
| IgG+-wells [%] | 22 | 12 | 7 | 6 |

### Selection steps prior to co-cultivation:

B-cells producing antibodies that specifically bind an antigen can be enriched from peripheral blood mononuclear cells (PBMCs). In one embodiment the PBMCs are depleted of macrophages. This is advantageous as outlined below, e.g. as in one embodiment for B-cells obtained from rabbit, for the co-cultivation step.

Macrophages can be depleted from PBMCs by adhesion to the surface of the cell culture plate (see preincubation step).

It has been found that incubating the population of B-cells at 37 °C for one hour in EL-4 B5 medium prior to the single cell depositing increases the total number of antibody secreting cells after the single cell depositing compared to a single cell depositing directly after the isolation and optional enrichment of the population of B-cells from the blood of an experimental animal (example rabbit, see Tables 2a and 2b).

**Table 2a: IgG positive wells with and without one hour incubation in EL-4 B5 medium prior to single cell depositing of all cells.**

| **rbIgG ELISA** | **fresh PBMCs (∅ 100-20 cells)** | **PBLs after incubation* (∅ 50-10 cells)** |
|---|---|---|
| rbIgG+ wells [n] | 40 | 108 |
| rbIgG+ wells [% total wells] | 28 | 75 |

| | | |
|---|---|---|
| * depleted of macrophages and monocytes | | |

**Table 2b: IgG positive wells with and without one hour incubation in EL-4 B5 medium prior to single cell depositing of B-cells.**

| **rbIgG ELISA** | **single B-cells from fresh blood** | **single B-cells from blood, 1h incubated** | **single B-cells from fresh spleen** | **single B-cells from spleen, 1h incubated** |
|---|---|---|---|---|
| rbIgG⁺ wells [n] | 2 | 55 | 6 | 52 |
| rbIgG⁺ wells [% total wells] | 2 | 33 | 7 | 31 |

In one embodiment of the method as reported herein the cells are obtained from a protein-immunized animal and depleted of macrophages.

Cells not producing an antibody binding the antigen or, likewise, cells producing an antibody binding to the antigen with can be reduced or enriched, respectively, by using a panning approach. Therein a binding partner is presented attached to a surface and cells binding thereto are selectively enriched in the cell population in case the bound cells are processed further or reduced in the cell population in case the cells remaining in solution are processed further.

**Table 3: Enrichment of B-cells secreting an antigen-specific antibody by panning with the respective antigen.**

| **protein antigen** | **without panning** | **with panning using the antigen** |
|---|---|---|
| total wells [n] | 4284 | 2113 |
| antigen specific IgG+ wells [n] | 235 | 419 |
| antigen specific IgG+ wells [% total wells] | 5 | 20 |
| **small molecule antigen** | **without panning** | **with panning using the small molecule** |
| total wells [n] | 336 | 336 |
| small molecule IgG+ wells [n] | 2 | 115 |
| small molecule IgG+ wells [% total wells] | 1 | 34 |

The method as reported herein comprises in one embodiment prior to the single cell depositing a selecting step in which B-cells producing specific and/or non-cross-reactive antibodies are selected based on cell surface markers and fluorescence activated cell sorting/gating. In one embodiment mature B-cells are sorted/enriched/selected. For selection of B-cells from different experimental animal species different cell surface markers can be used. It has been found that many of the available cell surface markers, either individually or in combination, do not provide for a suitable labeling.

With the labeling of non-target cell populations and non-specifically binding lymphocytes it is possible to selectively deplete these cells. In this depletion step only a non total depletion can be achieved. Albeit the depletion is not quantitative it provides for an advantage in the succeeding fluorescence labeling of the remaining cells as the number of interfering cells can be reduced or even minimized. By a single cell depositing of mature B-cells (memory B-cells, affinity matured plasmablasts and plasma cells) by fluorescence activated cell sorting using the labeling as outlined below a higher number of IgG⁺-wells in the co-cultivation step can be obtained.

The term "labeling" denotes the presence or absence of a surface marker which can be determined by the addition of a specifically binding and labeled anti-surface marker antibody. Thus, the presence of a surface marker is determined e.g. by the occurrence of a fluorescence whereas the absence of a surface marker is determined by the absence of the occurrence of a fluorescence each after incubation with the respective specifically binding and labeled anti-surface marker antibody.

Different cell populations can be labeled by using different surface markers such as CD3⁺-cells (T-cells), CD19⁺-cells (B-cells), IgM⁺-cells (mature naive B-cells), IgG⁺-celis (mature B-cells), CD38⁺-cells (e.g. plasmablasts), and IgG⁺CD38⁺-cells (pre-plasma cells).

As reported herein an immuno-fluorescence labeling for selection of mature IgG⁺-B-cells, such as memory B-cells, plasmablasts and plasma cells, has been developed. For a selection or enrichment of B-cells the cells are either singles labeled or double labeled. Also required is a labeling that results in about 0.1 % to 2.5 % of labeled cells of the total cell population. In one embodiment B-cells are deposited as single cells selected by the labeling of surface molecules present on 0.1 % to 2.5 % of all B-cells, in another embodiment on 0.3 % to 1.5 % of all B-cells, in a further embodiment on 0.5 % to 1 % of all B-cells.

Of IgG⁺-B-cells within the PBMC population 0.5 % - 1 % can be doubly labeled as IgG⁺CD19⁺-cells, IgG⁺CD38⁺-cells, and IgG⁺CD268⁺-cells. Thus, in one embodiment IgG⁺CD19⁺-B-cells, IgG⁺CD38⁺-B-cells or IgG⁺CD268⁺-B-cells are deposited as single cells.

Of IgG⁻-B-cells within the PBMC population 0.5 % - 1 % can be doubly labeled as IgG⁻CD138⁺-cells. Thus, in one embodiment IgG⁻CD138⁺-B-celis are deposited as single cells.

The labeling of CD27⁺CD138⁺-cells or CD3⁻CD27⁺-cells results in about 1.5 % of the cells of the cell population to be labeled, respectively. Thus, in one embodiment CD27⁺CD138⁺-B-cells or CD3⁻CD27⁺-B-cells are deposited as single cells.

Of IgG⁺-hamster-B-cells within the PBMC population 0.6 % ± 0.1 % can be doubly labeled as IgG⁺IgM⁻-hamster-B-cells. Thus, IgG⁺IgM⁻-hamster-B-cells are deposited as single cells.

In one embodiment IgG⁻CD138⁺-B-cells are deposited as single cells from the B-cells obtained from an immunized animal. In one embodiment IgG⁺CD19⁺-B-cells are deposited as single cells from the B-cells obtained from a non-immunized animal. In another embodiment IgG⁺IgM⁻-B-cells are deposited as single cells from the B-cells obtained from a non-immunized or immunized animal. IgG⁺CD19⁺-murine-B-cells are deposited as single cells. This selection step results in the highest yield of IgG⁺-wells in the succeeding co-cultivation step. IgG-CD138⁺-murine-B-cells are deposited as single cells. Therewith cells producing the highest amount of B-cell clones in the first place and secondly the highest concentration of IgG are selected (see Table 5). IgG⁺CD19⁺-murine-B-cells and IgG⁻CD138⁺-murine-B-cells are deposited as single cells.

IgG⁺-murine-B-cells can be labeled with the anti-mouse-IgG-antibody 227 (Ab 227), IgG⁺-hamster-B-cells can be labeled with the anti-hamster-IgG-antibody 213 (AB 213) and/or anti-hamster-IgG-antibody 225 (AB 225), and rabbit B-cells can be labeled with the anti-IgG-antibody 184 (see Table 4).

**Table 4: Immunofluorescence labeling of B-cells - the table present the average labeled fraction of the population of murine B-cells (A-E), hamster B-cells (F-H) and rabbit B-cells (I-J).**

| | **Single IgG labeling** | **IgG+CD19 labeling** | **IgG+IgM labeling** |
|---|---|---|---|
| A | IgG⁺ | - | IgG⁺IgM⁺ |
| | AB 185 PE | | AB 185 PE, AB 219 APC |
| | 17 % ± 3 % n=4 | | 12 % n= 1 |
| B | IgG⁺ | IgG⁺CD19⁺ | IgG⁺IgM⁺ |
| | AB 215 APC | AB 215 APC, AB 218 PE | AB 215 APC, AB 200 PE |
| | 12 % ± 3 % n=5 | 11 % n=1 | 14 % n= 1 |
| C | IgG⁺ | IgG⁺CD19⁺ | IgG⁺IgM⁺ |
| | AB 217 FITC | AB 217 FITC, AB 218 PE | AB 217 FITC, AB 200 PE |
| | 17 % ± 4 % n=7 | 10 % n=1 | 19 % n= 1 |
| D | IgG⁺ | IgG⁺CD19⁺ | IgG⁺IgM⁺ |
| | AB 222 FITC | AB 222 FITC, AB 218 PE | AB 222 FITC, AB 200 PE |
| | 18 % ± 2 % n=3 | 15 % n=1 | 14 % n= 1 |
| E | IgG⁺ | IgG⁺CD19⁺ | IgG⁺IgM⁺ |
| | AB 227 FITC | AB 227 FITC, AB 218 PE | AB 227 FITC, AB 200 PE |
| | 0.8 % ± 0.3 % n= 13 | 0.5 % n=1 | 0.2 % n= 1 |
| F | IgG⁺ | no B-cell marker known | IgG⁺IgM⁺ |
| | AB 212 FITC | | AB 212 FITC, AB 223 APC |
| | 43 % ± 6 % n=7 | | 43 % n= 1 |
| G | IgG⁺ | no B-cell marker known | IgG⁺IgM⁺ |
| | AB 213 APC | | AB 213 APC, AB 224 FITC |
| | 0.9 % ± 0.4 % n=27 | | 0.07 % n= 1 |
| H | IgG⁺ | no B-cell marker known | IgG⁺IgM⁺ |
| | AB 225 PE | | AB 225 PE, AB 224 FITC |
| | 17 % ± 3 % n=5 | | 0.7 % n= 1 |
| I | IgG⁺ | - | - |
| | AB 120 PE | | |
| | > 10 % | | |
| J | IgG⁺ | - | - |
| | AB 184 FITC | | |
| | 0.3 - 2 % | | |

| | | | |
|---|---|---|---|
| AB 120 - goat anti-rabbit IgG-antibody Southern Biotech 4030-09 AB 184 - goat anti-rabbit IgG Fc-antibody AbDSerotech STAR121F AB 185 - goat anti-mouse IgG-antibody Caltag M35004-3 AB 200 - goat anti-mouse IgM-antibody Invitrogen M31504 AB 212 - goat anti-hamster IgG-antibody AbDSerotech STAR79F AB 213 - mouse anti-hamster IgG-antibody Becton Dickinson 554010 AB 215 - goat anti-mouse IgG-antibody Sigma B 0529 AB 217 - goat anti-mouse IgG-antibody AbDSerotech STAR120F AB 218 - rat anti-mouse CD 19-antibody Abcam ab22480 AB 219 - goat anti-mouse IgM-antibody Rockland 710-1607 AB 222 - goat anti-mouse IgG-antibody Abcam ab7064 AB 223 - mouse anti-hamster IgM-antibodyBecton Dickinson 554035 AB 224 - mouse anti-hamster IgM-antibody Becton Dickinson 554033 AB 225 - mouse anti-hamster IgG-antibody Becton Dickinson 554056 AB 227 - goat anti-mouse IgG-antibody Sigma F 8264 PE: Phycoerythrin APC: Allophycocyanin FITC: Fluorescein isothiocyanate | | | |

It has to be pointed out that not all antibodies could be used for the labeling due to their low or non existing specificity.

Murine-B-cells can be labeled with the anti-IgG-antibody 227, hamster-B-cells can be labeled with the anti-IgG-antibody 213.

IgG⁺CD19⁺-murine-B-cells can be labeled with antibody 227 and antibody 218,
IgG⁺IgM⁻-murine-B-cells can be labeled with antibody 227 and antibody 219,
IgG⁺IgM⁻-hamster-B-cells can be labeled with antibody 213 and antibody 224,
IgG⁺-rabbit-B-cells can be labeled with antibody 184,
IgG⁺IgM⁻-rabbit-B-cells can be labeled with antibody 184 and antibody 254 and SA 263,
IgG⁺CD138⁺-rabbit-B-cells can be labeled with antibody 259 and antibody 256.

Murine B-cells can be labeled with the anti-CD27 antibody 235 or 236 (AB 235, AB 236), the anti-CD38 antibody 192 (AB 192), the anti-CD138 antibody 233 (AB 233) and the anti-CD268 antibody 246 (AB 246).

**Table 5: Immuno fluorescence labeling for the determination of mature mouse- (A-J), hamster- (K) and rabbit (L-N)-B-cells.**

| **labeling** | **Immuno fluorescence labeling for sorting of B-cells** | **Percentage of all viable cells %** |
|---|---|---|
| A | IgG⁺CD19⁺ - AB 227 FITC, AB 218 PE | 0.5 ± 0.2 n=14 |
| B | IgG⁺CD38⁺ - AB 227 FITC, AB 192 PE | 0.8 ± 0.5 n= 9 |
| C | IgG⁺CD138⁺ - AB 227 FITC, AB 233 PE | 0.06 ± 0.07 n= 6 |
| D | IgG⁻CD138⁺ - AB 227 FITC, AB 233 PE | 0.6 ± 0.5 n=6 |
| E | IgG⁺CD27⁺ - AB 227 FITC, AB 235 PE | 0.1 ± 0.1 n= 8 |
| F | CD27⁺CD138⁺ - AB 236 A647, AB 233 PE | 1.5 ± 0.5 n= 2 |
| G | CD27⁺IgG⁺CD3⁻ - AB 235 PE, AB 227 FITC, AB 241 A647 | 0.10 ± 0.04 n= 3 |
| H | CD3⁻CD27⁺ - AB 189 FITC, AB 235 PE | 1.33 n= 1 |
| I | IgG⁺CD268⁺ - AB 227 FITC, AB 246 A647 | 0.8 n= 1 |
| J | CD38⁺CD3⁻ - AB 192 PE, AB 189 FITC | 12 ± 7 n= 2 |
| K | IgG⁺IgM⁻ - AB 213 A647, AB 224 FITC | 0.6 ± 0.1 n= 15 |
| L | IgG⁺ - AB 184 FITC | 0.6 ± 0.2, n= 5 |
| M | IgG⁺IgM⁻ - AB 184 FITC, AB 254 Biotin, SA 263 PE | 0.4 ± 0.2, n=2 |
| N | IgG⁺CD138⁺ - AB 259, AB 256 PE | 0.3 ± 0.1, n= 5 |

| | | |
|---|---|---|
| AB 184 - goat anti-rabbit IgG-antibody AbD Serotec STAR121F AB 189 - hamster anti-mouse CD3-antibody Becton Dickinson 553062 AB 192 - rat anti-mouse CD38-antibody Becton Dickinson 553764 AB 213 - mouse anti-hamster IgG-antibody Becton Dickinson 554010 AB 218 - rat anti-mouse CD19-antibody Abcam ab22480 AB 224 - mouse anti-hamster IgM-antibody Becton Dickinson 554033 AB 227 - goat anti-mouse IgG-antibody Sigma F 8264 AB 233 - rat anti-mouse CD138-antibody Becton Dickinson 553714 AB 235 - hamster anti-mouse CD27-antibody Becton Dickinson 558754 AB 236 - hamster anti-mouse CD27-antibody Becton Dickinson 558753 AB 241 - hamster anti-mouse CD3-antibody Becton Dickinson 553060 AB 246 - rat anti-mouse BAFF-R-antibody eBioscience 51-5943 AB 254 - mouse anti-rabbit IgM-antibody Becton Dickinson custom made AB 256 - goat anti-rat IgG-antibody Southern Biotech 3030-09 AB 259 - rat anti-rabbit CD138-antibody Roche Glycart AG SA 263 - Streptavidin Invitrogen S866 A647: Alexa Fluor® 647 FITC: Fluorescein isothiocyanate | | |

In one embodiment the method comprises the step of depleting of macrophages and enriching of B-cells secreting antibody specifically binding a target antigen.

### Single cell depositing:

The method as reported herein comprises the step of depositing the B-cells as single cells. In one embodiment the depositing as single cells is by fluorescence activated cell sorting (FACS). The labeling required for the FACS single cell depositing can be carried out as reported in the previous section.

In one embodiment specifically labeled B-cells are deposited as single cells. In a further embodiment the labeling is a labeling of cell surface markers with fluorescence labeled antibodies. In another embodiment the method as reported herein provides for monoclonal antibodies. In one embodiment mature B-cells are deposited as single cells.

It has also been found that an additional centrifugation step after the single cell depositing and prior to the co-cultivation provides for an increased number of antibody secreting cells (example experimental animal with human immunoglobulin locus, see Table 6).

**Table 6: IgG positive wells with and without centrifugation step after single cell depositing.**

| **huCk ELISA** | **with centrifugation step** | **without centrifugation step** |
|---|---|---|
| huCk⁺ wells [n] | 9 | 1 |
| huCk⁺ wells [% total wells] | 13 | 1 |
| huCk conc. of all huCk⁺ wells [average ng/ml] | 76.4 | 9.7 |

Thus, in one embodiment the method as reported herein comprises the step of incubating the B-cells in EL-4 B5 medium for about one hour prior to single cell depositing. In another embodiment the method comprises the step of centrifuging the single deposited cells prior to the co-cultivation. In one embodiment the centrifuging is for 5 min. at 300 x g.

### Co-cultivation:

The co-cultivation step with feeder cells can be preceded and also succeeded by a number of additional steps.

In one embodiment the single deposited B-cells are co-cultivated with feeder cells in the presence of a feeder mix. In another embodiment the B-cells are co-cultivated with murine EL-4 B5 feeder cells. By suitable immuno fluorescence labeling as outlined above an increase in the yield in the co-cultivation step (number of IgG⁺-wells as well as IgG-concentration) and also an enrichment or isolation of mature IgG⁺-B-cell from PBMCs can be achieved.

With the single cell depositing of IgG⁺CD19⁺- and/or IgG⁺CD38⁺-B-cells from freshly isolated PBMCs the highest number of IgG⁺-wells can be obtained. With the single cell depositing of IgG⁺CD19⁺-, IgG⁺CD38⁺- and/or IgG-CD138⁺-B-cells after the depletion of macrophages or KHL-specific cells good results can be obtained. With the single cell depositing of IgG⁺CD 19⁺-, IgG⁺CD38+- and/or IgG-CD138⁺-B-cells after the depletion of antigen-specific B-cells the best results can be obtained. Thus, in one embodiment IgG⁺CD19⁺-, IgG⁺CD38⁺- and/or IgG⁻ CD138⁺-B-cells are deposited as single cells.

It has been found that by single cell depositing based on a labeling as outlined above results in the highest fraction of IgG⁺-wells and in the wells with the highest IgG-concentration. Thus, IgG⁺CD19⁺- and/or IgG⁻CD138⁺-murine-B-cells are deposited as single cells. IgG⁺IgM⁻-hamster-B-cells are deposited as single cells. In one embodiment IgG⁺-, and/or IgG⁺CD138⁺-, and/or CD138⁺- and/or IgG⁺IgM⁻-rabbit-B-cells are deposited as single cells.

**Table 7: Yield in the co-cultivation depending on the immuno fluorescence labeling.**

| **labeling** | | **n_{ges}** | **IgG⁺-wells of n_{ges} (%)** | | | **IgG-concentration (ng/ml)** | | |
|---|---|---|---|---|---|---|---|---|
| | | | Isol. | Abr. | Anr. | Isol. | Abr. | Anr. |
| mouse | IgG⁺CD19⁺ | 356/ 356/324 | 45 | 50 | 37 | 68 | 46 | 42 |
| | IgG⁺ | -/144/144 | - | 32 | 7 | - | 34 | 31 |
| | IgG⁺CD38⁺ | 72/190/190 | 36 | 41 | 43 | 37 | 26 | 27 |
| | IgG⁺ CD138⁺ | 72/72/72 | 3 | 13 | 12 | 22 | 59 | 43 |

| **labeling** | | **n_{ges}** | **IgG⁺-wells of n_{ges} (%)** | | | **IgG-concentration (ng/ml)** | | |
|---|---|---|---|---|---|---|---|---|
| | IgG⁻ CD138⁺ | 36/108/48 | 19 | 52 | 37 | 55 | 31 | 51 |
| | IgG⁺CD27⁺ | 64/64/64 | 4 | 28 | 20 | 102 | 54 | 32 |
| | CD27⁺CD1 38⁺ | -/32/- | - | 6 | - | - | 135 | - |
| | CD27⁺IgG⁺ CD3⁻ | 72/72/72 | 14 | 0 | 14 | 4 | 0 | 0 |
| | CD3⁻CD27⁺ | -/32/- | - | 13 | - | - | 29 | - |
| hamster | IgG⁺ CD268⁺ | -/72/- | - | 35 | - | - | 93 | - |
| | IgG⁺IgM⁻ | -/216/216 | - | 17 | 22 | - | 78 | 93 |
| | IgG⁺ | -/216/216 | - | 10 | 35 | 1 | 71 | 64 |
| rabbit | IgG⁺ | -/1512/1307 | - | 33 | 28 | - | 59 | 60 |
| | IgG⁺IgM⁻ | -/76/- | - | 29 | - | - | 5 | - |
| | CD138⁺ | -/2016/- | - | 14 | - | - | 16 | - |
| | IgG⁺CD138⁺ | -/168/- | - | 37 | - | - | 64 | - |

For murine B-cells with the single cell depositing of IgG⁺CD19⁺-cells after each enrichment and/or depletion step the highest number of IgG⁺-wells after co-cultivation can be obtained. Alternatively, with the single cell depositing of IgG⁻ CD138⁺-cells wells with the best IgG-concentration in the supernatant can be obtained. The single cell depositing of IgG⁻CD138⁺-cells can be used for B-cells from immunized animals. The single cell depositing of IgG⁺CD19⁺-cells can be used for B-cells from non-immunized animals. The single cell depositing of IgG⁺IgM⁻-cells can be used for hamster-B-cells of immunized and non-immunized animals. The single cell depositing of IgG⁺-, and/or IgG⁺CD138⁺-, and/or CD 138⁺-and/or IgG⁺IgM⁻-B-cells can be used for rabbit-B-cells.

The immuno fluorescence labeling used for B-cells obtained from the blood of an experimental animal can also be used for the labeling of B-cells obtained from the spleen and other immunological organs of an experimental animal, such as mouse, hamster and rabbit. For mouse B-cells the fraction of IgG⁺-B-cells from spleen was about 0.8 % compared to 0.4 % for IgG⁺CD19⁺-cells. For hamster B-cells the respective numbers are 1.9 % and 0.5 % IgG⁺IgM—cells. For rabbit-blood 0.2 % of IgG⁺-cells were found after depletion of macrophages. Peyer'sche plaques from rabbit showed 0.4 % of IgG⁺-cells and spleen showed 0.3 % of IgG⁺-cells after depletion of macrophages.

With the method as reported herein after about seven (7) days, i.e. of 5, 6, 7, or 8 days, especially of 7 or 8 days, of co-cultivation antibody concentrations of from about 30 ng/ml up to 15 µg/ml or more can be obtained (average value about 500 ng/ml). With the thereby provided amount of antibody a higher number of different analyses can be performed in order to characterize the antibody, e.g. regarding binding specificity, in more detail. With the improved characterization of the antibody at this early stage in the screening / selection process it is possible to reduce the number of nucleic acid isolations and sequencing reactions that have to be performed. Additionally the B-cell clone provides an amount of mRNA encoding monoclonal light and heavy chain variable region allowing the use of degenerated PCR primer and obviates the requirement of highly specific primer. Also the required number of PCR cycles is reduced.

In one embodiment the feeder mix is a thymocyte cultivation supernatant.

Due to the origin of the feeder mix, which is derived from the supernatant of cultivated thymocytes (thymocyte cultivation supernatant - TSN), considerable batch to batch variations occur. In order to overcome this drawback a synthetic feeder mix consisting of synthetic components has been developed. A feeder mix consisting of IL-1β (interleukin-1beta), TNFα (tumor necrosis factor alpha), IL-2 (interleukin-2) and IL-10 (interleukin-10) is known from Zubler et al.

It is reported herein a synthetic feeder mix for the co-cultivation of single deposited B-cells and feeder cells. Also reported herein are B-cell-species-specific additives for the synthetic feeder mix for increasing the amount of secreted antibody by the respective B-cell. Concomitantly highly producing cells contain more mRNA which in turn facilitates the reverse transcription and sequencing of the encoding nucleic acid, e.g. with a redundant, non-specific primer set.

By the addition of SAC (Staphylococcus aureus strain Cowans cells) the number of antibody secreting B-cells and the average IgG-concentration in the supernatant after co-cultivation can be increased. It has been found that for the addition of SAC in the co-cultivation a concentration range can be defined as smaller as well as larger concentrations of SAC reduce the amount of secreted antibody.

**Table 8a: Results of a huCk ELISA or rbIgG ELISA of cell culture supernatants of B-cells obtained from an experimental animal with human IgG locus or a wildtype rabbit (NZW) co-cultivated with EL-4 B5 feeder cells and TSN as feeder mix with or without added SAC.**

| | | **TSN** | | **TSN+ SAC** | | | | | |
|---|---|---|---|---|---|---|---|---|---|
| huCk⁺ wells [n] | | 7 | | 45 | | | | | |
| huCk⁺ wells [% total wells] | | 5 | | 31 | | | | | |
| huCk conc. of all huCk+ wells [Ø ng/ml] | | 89.1 | | 41.0 | | | | | |
| | | SAC 1:5000 | | SAC 1:10000 | | SAC 1:20000 | | SAC 1:40000 | |
| rbIpG⁺ wells [n] | | 13 | | 15 | | 27 | | 30 | |
| rbIgG⁺ wells [% total wells] | | 15 | | 18 | | 32 | | 36 | |
| rbIgG conc. of all rbIgG+ wells [Ø ng/ml] | | 149.0 | | 159.1 | | 233.7 | | 197.2 | |
| | | | | | | | | | |

| | **w/o** | | **SAC 1:20000** | | **SAC 1:50000** | | **SAC 1:100000** | | **SAC 1:150000** |
|---|---|---|---|---|---|---|---|---|---|
| rbIgG+ wells [n] | 12 | | 75 | | 93 | | 92 | | 72 |
| rbIgG+ wells [% total wells] | 5 | | 30 | | 37 | | 37 | | 29 |
| rbIgG conc. of all rbIgG+ wells [Ø ng/ml] | 199 | | 665 | | 742 | | 774 | | 668 |

It can be seen that a SAC ratio of from 1:20000 to 1: 150000 provides for an increased number of IgG⁺-wells, whereby the ratio of from 1:50000 to 1:100000 shows the highest numbers.

It has been observed that by the addition of SAC to the feeder-mix the co-cultivation of B-cells was surprisingly changed in such a way that only single deposited B-cells have a benefit in growth, whereas B-cell growth was inhibited when using a PBL (e.g. B cells and endogenous T cells) mixture for co-cultivation.

**Table 8b: Results of a huCk ELISA or rbIgG ELISA of cell culture supernatants of PBLs and single deposited B-cells co-cultivated with EL-4 B5 feeder cells and TSN as feeder mix with added SAC.**

| **rbIgG ELISA** | **PBLs* (30 cells)** | **single deposited rbIgG+ B-cell** |
|---|---|---|
| rbIgG⁺ wells [n] | 8 | 104 |
| rbIgG⁺ wells [% total wells] | 6 | 58 |
| rbIgG conc. of all huCk⁺ wells [average ng/ml] | 55.0 | 129.2 |

| | | |
|---|---|---|
| * depleted of macrophages | | |

Further data obtained with different feeder mixes is presented in the following Tables 9 and 10.

In one embodiment the feeder mix for the co-cultivation of B-cells comprises IL-1β, TNFα, IL-2, IL-10 and IL-21 (interleukin-21). In one embodiment the feeder mix for the co-cultivation of B-cells comprises IL-1β, TNFα, IL-2, IL-10 and SAC. In another embodiment IL-1β, TNFα, IL-2, IL-10 and IL-21 are recombinant murine IL-1β, TNFα, IL-2, IL-10 and IL-21.

The feeder mix for the co-cultivation of murine B-cells comprises IL-1ß, IL-2, IL-10, TNF-α and BAFF. BAFF is added at a concentration of 5 ng/ml.

The feeder mix for the co-cultivation of hamster B-cells comprises IL-1ß, IL-2, IL-10, TNF-α, IL-6 and SAC. IL-6 is added at a concentration of 10 ng/ml. SAC is added at a 1:75,000 ratio.

**Table 9: Results of an rbIgG ELISA of cell culture supernatants of B-cells co-cultivated with EL-4 B5 feeder cells and different synthetic feeder mixes comprising recombinant murine substances in different combinations.**

| | **rabbit TSN, SAC** | **IL-6, IL-1β, TNFα, IL-2, IL-10** | **IL-6, TNFα, IL-2, IL-10** | **IL-6, IL-1β, IL-2, IL-10** | **IL-6, IL-1β, TNFα, IL-2** | **IL-6, IL-1β, TNFα, IL-10** | **IL-1β, TNFα, IL-2, IL-10** |
|---|---|---|---|---|---|---|---|
| rbIgG⁺ wells [n] | 37 | 24 | 12 | 16 | 18 | 23 | 24 |
| rbIgG⁺ wells [% total wells] | 51 | 33 | 17 | 22 | 25 | 32 | 33 |

**Table 10: IgG⁺-wells of cell culture supernatants of B-cells co-cultivated with EL-4 B5 feeder cells and TSN or a feeder mix comprising recombinant murine substances and SAC (rb = rabbit, m = mouse).**

| rbIgG⁺ wells [n] | **TSN + SAC** | **IL-1β, TNFα, IL-2, IL-10 + SAC** |
|---|---|---|
| pure | 64 | 55 |
| + mIL21 | 22 | 25 |
| + mIL10 | 78 | 61 |
| + mIL21 + mIL10 | 57 | 93 |
| rbIgG⁺ wells [% total wells] | | |
| pure | 25 | 22 |
| + mIL21 | 9 | 10 |
| + mIL10 | 31 | 24 |
| + mIL21 + mIL10 | 23 | 37 |
| rbIgG conc. of all rbIgG+ wells [∅ ng/ml] | | |
| pure | 312.3 | 662.3 |
| + mIL21 | 263.7 | 541.1 |
| + mIL10 | 553.0 | 522.3 |
| + mIL21 + mIL10 | 422.6 | 307.5 |

A co-cultivation of feeder cells and murine B-cells without IL-2, without IL-10 as well as without IL-2 and IL-10 results in an increase in the yield of IgG⁺-wells albeit the IgG-concentration is reduced. Without TNFα the IgG-concentration is also reduced. Without IL-1ß no IgG can be found in the supernatant.

A co-cultivation of hamster B-cells without IL-2 and without IL-10, respectively, results in IgG⁺-wells with detectable IgG-concentration. In contrast thereto in a co-cultivation without IL-2 and IL-10 almost no B-cell growth can be detected. In the absence of TNF-α or IL-1ß no IgG-secretion can be determined.

In the presence of EL-4 B5 feeder cells at least IL-1ß and TNFα are required for the co-cultivation of mouse, hamster and rabbit B-cells. IL-2 and IL-10 can be omitted for the co-cultivation of murine cells. Hamster B-cells can be cultivated in the absence of either IL-2 or IL-10. Rabbit B-cells can be cultivated in the absence of either IL-2 or IL-10 or IL-6.

For murine and hamster B-cells the addition of IL-4 to the feeder mix increases the number of IgG⁺-wells as well as the IgG-concentration in the supernatant. Thus, the feeder mix for the co-cultivation of murine- or hamster-B-cells comprises IL-4.

The addition of IL-6 to the feeder mix for the co-cultivation of murine-B-cells or hamster-B-cells results in an increased number of IgG⁺-wells or increased IgG-concentration, respectively. Thus, the feeder mix for the co-cultivation of murine-B-cells or hamster-B-cells comprises IL-6. In one embodiment the IL-6 is added at a concentration of 50 ng/ml. In one embodiment IL-6 is added at a concentration of 10 ng/ml, if high IgG-concentration is required. In one embodiment the addition of IL-6 is after three days of co-cultivation of the selected B-cells and EL-4 B5 cells. The addition of an inhibitor of a certain potassium channel (= PAP-1, 5-(4-phenoxybutoxy) psoralene) surprisingly increases the rbIgG secretion of B-cells in a concentration dependent manner without decreasing the number of B-cells. Usually a cytokine which induced rbIgG productivity can be correlated with a decrease of the overall number of B-cell clones. This was not the case with PAP-1.

**Table 11: Results of an rbIgG ELISA of cell culture supernatants of B-cells co-cultivated with EL-4 B5 feeder cells in the presence of TSN and SAC (=w/o) and different concentrations of PAP-1. DMSO: solvent for PAP-1 (1 µM).**

| | **w/o** | **0.01 µM** | **0.1 µM** | **1 µM** | **10 µM** | **DMSO** |
|---|---|---|---|---|---|---|
| rbIgG+ wells [n] | 53 | 72 | 69 | 93 | 80 | 76 |
| rbIgG+ wells [% total wells] | 21 | 29 | 27 | 37 | 32 | 30 |
| rbIgG conc. of all huCk⁺ wells [average ng/ml] | 195.8 | 289.0 | 452.9 | 579.5 | 890.7 | 225.3 |

With a TSN concentration of 7.5 % the highest IgG concentration in the supernatant can be obtained.

**Table 12: Influence of TSN on co-cultivation. A TSN concentration of 7.5% is advantageous in terms of B-cell growth and productivity**

| | **5% TSN** | **7.5% TSN** | **10% TSN** |
|---|---|---|---|
| rbIgG+ wells [n] | 71 | 71 | 81 |
| rbIgG+ wells [% total wells] | 28 | 28 | 32 |
| rbIgG conc. of all rbIgG+ wells [∅ ng/ml] | 246 | 512 | 372 |

With a number of 30,000 feeder cells per well of a 96-well plate the highest number of IgG⁺-wells in combination with IgG concentration in the supernatant can be obtained.

**Table 13: Influence of the amount of EL-4 B5 feeder cells on co-cultivation.**

| | **20000** | **22000** | **24000** | **30000** | **35000** | **40000** |
|---|---|---|---|---|---|---|
| rbIgG+ wells [n] | 71 | 73 | 78 | 78 | 73 | 38 |
| rbIgG+ wells [% total wells] | 28 | 29 | 31 | 31 | 29 | 15 |
| rbIgG conc. of all rbIgG+ wells [∅ ng/ml] | 246 | 319 | 346 | 418 | 457 | 656 |

The co-cultivation is in one embodiment in polystyrene multi well plates with wells with a round bottom. The working volume of the wells is in one embodiment of 50 µl to 250 µl. In one embodiment the wells are coated at least partially with a non-fibrous substrate prepared from a blend of polymer plastic resin and amphipathic molecules, wherein the amphipathic molecule comprises a hydrophilic moiety and a hydrophobic region, wherein the hydrophobic regions are anchored within the substrate and the hydrophilic moieties are exposed on the substrate. In one embodiment the amphipathic molecules are chosen from alkylamine ethoxylated, poly (ethylene imine), octyldecamine or mixtures thereof (see e.g. EP 1 860 181).

### Characterization of co-cultivated cells:

For the (qualitative and quantitative) determination of secreted IgG after the co-cultivation generally all methods known to a person of skill in the art such as an ELISA can be used. In one embodiment an ELISA is used. For the determination of IgG secreted by murine B-cells an ELISA with the anti-IgG antibodies AB 216 (capture antibody) and AB 215 (tracer antibody) is used. For the determination of IgG secreted by hamster B-cells an ELISA with the monoclonal antibodies AB 220 (capture antibody) and AB 213 (tracer antibody) is used.

Depending on the characterization results a B-cell clone can be obtained, i.e. selected. The term "clone" denotes a population of dividing and antibody secreting B-cells arising from/originating from a single B-cell. Thus, a B-cell clone produces a monoclonal antibody.

### Isolation of mRNA, cloning and sequencing:

From the B-cells the total mRNA can be isolated and transcribed in cDNA. With specific primers the cognate VH- and VL-region encoding nucleic acid can be amplified. With the sequencing of the therewith obtained nucleic acid it can be confirmed that the obtained antibodies are monoclonal antibodies in most cases (71-95 %). Also can be seen from the sequencing of the individual B-cells that almost no identical sequences are obtained. Thus, the method provides for highly diverse antibodies binding to the same antigen.

The primers used for the amplification of the VH-encoding nucleic acid can be used for cDNA obtained from cells from the NMRI-mouse, the Armenian Hamster, the Balb/c-mouse as well as the Syrian hamster and the rabbit.

In one embodiment the amino acid sequence is derived from the amplified VH-encoding nucleic acid and the exact start and end point is identified by locating the amino acid sequences of EVQL/QVQL to VSS (VH-region) and DIVM/DIQM to KLEIK (VL-region).

The term "antibody" denotes a protein consisting of one or more polypeptide chain(s) substantially encoded by immunoglobulin genes. The recognized immunoglobulin genes include the different constant region genes as well as the myriad immunoglobulin variable region genes. Immunoglobulins may exist in a variety of formats, including, for example, Fv, Fab, and F(ab)₂ as well as single chains (scFv), diabodies, monovalent, bivalent, trivalent or tetravalent forms, and also as bispecific, trispecific or tetraspecific form (e.g. Huston, J.S., et al., Proc. Natl. Acad. Sci. USA 85 (1988) 5879-5883; Bird, R.E., et al., Science 242 (1988) 423-426; in general, Hood et al., Immunology, Benjamin N.Y., 2nd edition (1984); and Hunkapiller, T. and Hood, L., Nature 323 (1986) 15-16).

Also reported herein is a method for producing an antibody comprising the following steps
a) providing a population of mature B-cells obtained from the blood of a rabbit,
b) staining IgG⁺-B-cells and/or CD138⁺-B-cells of the population of B-cells with at least one fluorescence dye (in one embodiment with one to three, or two to three fluorescence dyes),
c) incubating the B-cells at 37 °C for one hour in co-cultivation medium prior to depositing single cells of the stained population of B-cells in individual containers (in one embodiment is the container a well of a multi well plate),
d) cultivating the deposited individual B-cells in the presence of feeder cells and a feeder mix (in one embodiment the feeder cells are EL-4 B5 cells, in one embodiment the feeder mix is natural TSN, in one embodiment the feeder mix is a synthetic feeder mix),
e) determining the binding specificity of the antibodies secreted in the cultivation of the individual B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid in an expression cassette for the expression of an antibody,
h) introducing the nucleic acid in a cell,
i) cultivating the cell and recovering the antibody from the cell or the cell culture supernatant and thereby producing an antibody.

An "expression cassette" refers to a construct that contains the necessary regulatory elements, such as promoter and polyadenylation site, for expression of at least the contained nucleic acid in a cell.

The term "experimental animal" denotes a non-human mammal. In one embodiment the experimental animal is selected from rat, mouse, hamster, rabbit, non-human primates, sheep, dog, cow, chicken, amphibians, and reptiles.

The following examples and sequence listing are provided to aid the understanding of the present invention, the true scope of which is set forth in the appended claims.

### Examples

### Example 1

### Media and buffers:

Blocking buffer for ELISA comprises 1X PBS and 1% BSA.

Coating buffer for ELISA comprises 4.29g Na2CO3* 10 H2O and 2.93g NaHCO3 add water to a final volume of 1 liter, pH 9.6 adjusted with 2 N HCl.

Ethanol-solution for RNA isolation comprises 70 % Ethanol or 80 % Ethanol.

FACS-buffer for immuno fluorescence staining comprises 1X PBS and 0.1 % BSA.

IMDM-buffer for ELISA comprises 1X PBS, 5 % IMDM and 0.5 % BSA.

Incubation buffer 1 for ELISA comprises 1X PBS, 0.5 % CroteinC.

Incubation buffer 2 for ELISA comprises 1X PBS, 0.5 % CroteinC and 0.02 % Tween 20.

Incubation buffer 3 for ELISA comprises 1X PBS, 0.1 % BSA.

Incubation buffer 4 for ELISA comprises 1X PBS, 0.5 % BSA, 0.05 % Tween, PBS (10X), 0.01 M KH2PO4, 0.1 M Na2HP04, 1.37 M NaCl, 0.027 M KCl, pH 7.0.

PCR-buffer comprises 500 mM KCl, 15 mM MgCl2, 100 mM Tris/HCl, pH 9.0.

Wash buffer 1 for ELISA comprises 1X PBS, 0.05 % Tween 20.

Wash buffer 2 for ELISA comprises 1X PBS, 0.1 % Tween 20.

Wash buffer 3 for ELISA comprises water, 0.9 % NaCl, 0.05 % Tween 20.

EL-4 B5 medium comprises RPMI 1640, 10 % FCS, 1 %

Glutamin/Penicillin/Streptomycin-Mix, 2 % 100 mM sodium pyruvate, 1 % 1 M HEPES buffer.

### Example 2

### Animal care and immunization

The experimental animals were held according to the German animal protection law (TierSCHG) as well as according to the respective European guidelines. Mice and hamster were received at an age of from 6 to 8 weeks and were immunized prior to an age of 12 weeks. The antigen was at first applied together with complete Freud's adjuvant (CFA). Further applications were with incomplete Freud's adjuvant (IFA). The antigen containing emulsion was applied subcutaneously whereby the emulsion comprised an amount of from 50 to 100 µg antigen depending on the weight of the receiving experimental animal.

During the immunization serum antibody titer was determined with an antigen specific assay. At an antibody titer with an IC50 of 1:10000 the blood or the spleen of the immunized animal was removed. For reactivation of antigen specific B-cells 30 to 50 µg of the antigen was applied intravenously to the experimental animal three days prior to the removal of the blood or the spleen.

### Example 3

### Removal of organs, blood and macrophages

Blood from the experimental animal was obtained by punctuation of the retrobulberic vein.

Macrophages can be isolated from the obtained blood. From mice and hamster about 3*10⁵ macrophages can be obtained from each animal.

If a larger amount of macrophages were required cells from the intraperitoneal cavity of the experimental animal were removed. For this the animals have to be at least 3 months of age. For the removal 5 ml of EL-4 B5 medium with a temperature of 37 °C was applied to the intraperitoneal cavity. After knotting the cavity for 5 minutes the solution containing the cells was removed.

### Example 4

### Cultivation of kL-4 B5 cells

The frozen EL-4 B5 cells were thawed rapidly in a water bath at 37 °C and diluted with 10 ml EL-4 B5 medium. After centrifugation at 300 x g for 10 minutes the supernatant was discarded and the pellet resuspended in medium. After a further centrifugation step the supernatant was discarded again and the pellet was resuspended in 1 ml medium.

The EL-4 B5 cells were inoculated at a cell density of 3 x 10⁴ cells/ml in 175 m² cultivation flasks. Cell density was determined every second day and adjusted to 3 x 10⁴ cell/ml. The cells have a doubling time of approximately 12 hours and have to be cultivated at a cell density below 5 x 10⁵ cell/ml because with higher cell density the stimulatory properties of the cells are lost.

When the total cell number was about 1.5 x 10⁹ cells the medium was removed by centrifugation. Afterwards the cells were irradiated with 50 gray (5000 rad). After the determination of the viable cell number by trypan blue staining between 5 x 10⁶ and 1 x 10⁷ cells are aliquoted and frozen at -80 °C.

For co-cultivation the cells were thawed and washed twice with EL-4 B5 medium. For determination of the viable cell number the cell suspension is diluted 1:10 with 0.4 % (w/v) trypan blue solution and 10 µl of the mixture is transferred to a Neubauer counting chamber and cell number was counted.

### Example 5

### Density gradient centrifugation

The isolation of peripheral blood mononuclear cells (PBMCs) was effected by density gradient separation with Lympholyte® according to manufacturer's instructions A (Lympholyte®-mammal, cedarlane).

Withdrawn blood was diluted 2:1 with phosphate buffered saline (PBS). In a centrifuge vial the same volume of density separation medium was provided and the diluted blood is carefully added via the wall of the vial. The vial was centrifuged for 20 min. at 800 x g without braking. The lymphocytes were obtained from the white interim layer. The removed cells were supplemented with 10 ml PBS and centrifuged at 800 x g for 10 min. The supernatant was discarded and the pellet was resuspended, washed, centrifuged. The final pellet was resuspended in PBS.

### Example 6

### Hypotonic lysis of red blood cells

For disruption of red blood cells by hypotonic lysis an ammonium chloride solution (BD Lyse^{™}) was diluted 1:10 with water and added at a ratio of 1:16 to whole blood. For lysis of the red blood cells the mixture was incubated for 15 min. in the dark. For separation of cell debris from intact cells the solution was centrifuged for 10 min. at 800 x g. The supernatant was discarded, the pellet was resuspended in PBS, washed again, centrifuged and the pellet was resuspended in PBS.

### Example 7

### Preparation of cells from inner organs of an experimental animal

For the preparation of spleen and thymus cells the respective organ was dissected in a Petri dish and the cells were taken up in PBS. For removal of remaining tissue the cell suspension was filtered through a 100 µm sieve. For obtaining lymphocytes from spleen cells density gradient centrifugation was employed. For thymus cells no further enrichment step was required.

### Example 8

### Depletion of macrophages

The cell suspension was adjusted to a cell density of 2 x 10⁶ cells/ml and three milliliter each was added to a well of a 6-well multi well plate. The plate was incubated for 60 to 90 min. at 37 °C. Thereafter the lymphocyte containing supernatant was transferred to a centrifugation vial and centrifuged at 800 x g for 10 min. The pellet was resuspended in PBS.

### Example 9

### Depletion of KHL-specific B-cells

Four milliliter of a solution containing keyhole limpet haemocyanine (KLH) was incubated with coating buffer at a concentration of 2 µg/ml in the wells of a multi well plate over night at room temperature. Prior to the depletion step the supernatant was removed and the wells were washed twice with PBS. Afterwards the blood cells were adjusted to a cell density of 2 x 10⁶ cells/ml and 3 ml are added to each well of a multi well plate. Afterwards the multi well plate was incubated for 60 to 90 min. at 37 °C. The supernatant was transferred to a centrifugation vial and the wells are washed twice with PBS and the supernatants are combined in the centrifugation vial. The cells were pelleted by centrifugation at 800 x g for 10 min. and the pellet was resuspended in PBS.

### Example 10

### Enrichment of antigen-specific B-cells

The respective antigen was diluted with coating buffer to a final concentration of 2 µg/ml. 3 ml of this solution were added to the well of a 6-well multi well plate and incubated over night at room temperature. Prior to use the supernatant was removed and the wells were washed twice with PBS. The B-cell solution was adjusted to a concentration of 2 x 10⁶ cells/ml and 3 ml are added to each well of a 6-well multi well plate. The plate was incubated for 60 to 90 min. at 37 °C. The supernatant was removed and the wells were washed two to four times with PBS. For recovery of the antigen-specific B-cells 1 ml of a trypsin/EDTA-solution was added to the wells of the multi well plate and incubated for 10 to 15 min. at 37 °C. The incubation was stopped by addition of medium and the supernatant was transferred to a centrifugation vial. The wells were washed twice with PBS and the supernatants were combined with the other supernatants. The cells were pelleted by centrifugation for 10 min. at 800 x g. The pellet was resuspended in PBS.

### Example 11

### Co-cultivation of B-cells and EL-4 B5 cells

The co-cultivation was performed in 96-well multi well plates with round bottom. A basis solution comprising EL-4 B5 cells (1.6 x 10⁶ cells / 15.2 ml) and cytokines in EL-4 B5 medium was prepared. 200 µl of the basis solution was added to each well of the multi well plate. To each well a single B-cell was added by fluorescence activated cell sorting. After the addition of the B-cells the plate was centrifuged for 5 min. at 300 x g. The plate is incubated for seven days at 37 °C.

### Example 12

### Cultivation of T-cells

The T-cells were isolated from the thymus of 3-4 week old mice and hamster, respectively. The cells were centrifuged and immediately cultivated or frozen in aliquots of 3 x 10⁷ cells. The thymocytes were seeded with a minimum cell density of 5 x 10⁵ cells/ml of EL-4 B5 medium in 175 cm² culture flasks and incubated for 48 hours at 37 °C.

### Example 13

### Cultivation of macrophages

Macrophages were isolated from the internal cavity of mice and hamster, respectively, of an age of at least three months. The macrophages were cultivated in EL-4 B5 medium at a cell density of at least 1 x 10⁵ cells/ml in 175 cm² culture flasks for 1.5 hours at 37 °C. Afterwards the medium was removed and the attached macrophages were washed with warm EL-4 B5 medium and cultivated for 48 hours in 35 ml medium.

### Example 14

### Co-cultivation of T-cells and macrophages

T-cells and macrophages were cultivated for 48 hours in separate flasks. Prior to combining the cells the T-cells were centrifuged for 10 min. at 800 x g. The supernatant was discarded and the pellet was resuspended in 10 ml medium. The T-cells were adjusted to a minimal cell density of 5 x 10⁵ cells/ml. Per ml medium 10 pg phorbol-12-myristat-13-acetat (PMA) and 5 ng or 50 ng Phytohemagglutinin M (PHA-M) were added. Prior to combining the cultivation medium of the macrophages was removed and the cells were added to the T-cell suspension in fresh medium. After 36 hours of co-cultivation the cultivation medium was removed and was termed TSN solution. For removal of remaining cells the TSN solution was filtered through a 0.22 µm filter. The TSN solution was frozen at -80 °C in aliquots of 4 ml.

### Example 15

### Immunofluorescence staining

Depending on the number of cells to be stained the cells were provided in 100 µl medium (less than 10⁶ cells) or 200 µl medium (more than 10⁶ cells), respectively. The fluorescent labeled antibody was diluted with 5 % serum of the experimental animal and FACS buffer to a final volume of 100 µl or 200 µl, respectively. The reaction mixture was incubated on a roller rack for 40 min. at 4 °C. After the incubation the cells were washed twice at 300 x g for 5 min. The pellet was resuspended in 400 µl PBS and filtered through a 70 µm sieve. The filtered solution was transferred to a FACS-vial and directly before the FACS experiment dead cells were stained by addition of propidium iodide (6.25 µg/ml). If the labeled antibody was labeled with biotin the antibody was detected in a second step with streptavidin labeled Alexa Flour(R) 647 (antibody 197).

### Example 16

### Quantification of IgG

The 96-well multi well plate in which the co-cultivation was performed was centrifuged after seven days of co-cultivation at 300 x g for 5 min. 150 µl supernatant was removed and diluted at a ratio of 2:1 with PBS in a second 96-well multi well plate.

The ELISA was performed as outlined in Example 17.

The antibody was used at a concentration of 50 ng/ml. If the OD was or exceeded 1 after an incubation time of 5 min. a dilution series of from 0.8 to 108 ng/ml IgG was tested.

### Example 17

### Detection of antigen-specific IgG

Antibodies produced by single deposited and co-cultivated B-cells or from B-cells obtained from an immunized experimental animal can be characterized with respect to specific antigen binding. The ELISA was performed at room temperature and the ELISA-solution was incubated between the individual steps on a shaker at 20 x g. In the first step the antigen was bound to the wells of a 96-well multi well plate. If the antigen was a protein it had been diluted in coating buffer and applied directly to the plate. Peptide antigens were bound via the specific binding pair biotin/streptavidin. The wells of the multi well plate can be already coated with soluble CroteinC (CrC) by the manufacturer. If not, the wells were incubated after the immobilization of the antigen with 200 µl blocking buffer. After the incubation with 100 µl antigen solution per well (pre-coated multi well plate) or 200 µl blocking buffer, respectively, non-bound antigen or blocking buffer was removed by washing with wash buffer. The diluted B-cell supernatants were added in a volume of 100 µl per well and incubated. After the incubation the wells were washed. Afterwards the detection antibody was added in a volume of 100 µl per well. The antibody can be either conjugated to horseradish peroxidase or labeled with biotin. The detection antibody was determined with a streptavidin-horseradish peroxidase conjugate. After the incubation the multi well plate was washed and afterwards 50 µl of a substrate solution containing 3,3',5,5' tetramethyl benzidine (TMB) were added per well and incubated for a period as given in Table X. The enzymatic reaction was stopped by the addition of 50 µl sulfuric acid and the optical density was determined at 450 nm and 680 nm with a photometer (Rainbow Thermo ELISA Reader) and the Xread plus-software.

### Example 18

### Isolation of ribonucleic acid (RNA)

The cells from which the RNA had to be isolated were at first pelleted by centrifugation. The cell pellet was lysed by the addition of 100 µl RLT-buffer with 10 µl/ml beta-mercaptoethanol. The cells were resuspended by multiple mixing with a pipette. The solution was transferred to a well of a multi well plate. The plate was shortly shock at 200 x g and frozen at -20 °C.

The isolation of the RNA was performed with the RNeasy® Kit (Qiagen, Hilden, Germany) according to the manufacturer's instructions.

### Example 19

### Reverse transcription polymerase chain reaction

The reverse transcription was carried out in a volume of 20 µl. For each reaction a control was performed with and without reverse transcriptase. Per reaction 1 µl dNTP (each at 10 mM), 0.4 µl oligo(dT)₁₂₋₁₈ (0.2 µg) and 0.6 µl random hexamer (0.03 µg) were pre-mixed and added to 8.5 µl RNA in H2O. The reaction mixture was incubated for 5 min. at 65 °C and directly afterwards transferred to ice. Thereafter 2 µl RT-buffer (10 x), 4 µl MgCl2 (25 mM), 2 µl DTT (0.1 M) and 1 µl RNAse Out^{™} (40 units) were pre-mixed and added to the ice cold reaction mixture. After an incubation time of 2 min. at room temperature 0.5 µl Superscript^{™} II reverse transcriptase (25 units) were added. The reaction mixture was incubated for 10 min. at room temperature.

The translation was carried out for 50 min. at 42 °C. After the translation the reverse transcriptase was inactivated by incubation for 15 min. at 70 °C. The cDNA was stored at -20 °C.

### Example 20

### Polymerase chain reaction

The polymerase chain reaction was carried out with the Taq PCR Core Kit (Qiagen, Hilden, Germany) according to the manufacturer's instructions. The PCR was carried out in a volume of 20 µl. The samples were transferred to the Mastercyler® at a temperature of 95°C.

### Example 21

### Sequencing

All sequences were determined by SequiServe (Vaterstetten, Germany).

### Example 22

### Panning on antigen

### a) Coating of plates

### Biotin/Streptavidin:

Sterile streptavidin-coated 6-well plates (cell culture grade) were incubated with biotinylated antigen at a concentration of 0.5 - 2 µg/ml in PBS at room temperature for one hour. Plates were washed in sterile PBS three times before use.

### Covalently bound protein:

Cell culture 6-well plates were coated with 2 µg/ml protein in carbonate buffer (0.1 M sodium bicarbonate, 34 mM disodium hydrogen carbonate, pH 9.55) over night at 4 °C. Plates were washed in sterile PBS three times before use.

### b) Panning of B-cells on peptides

6-well tissue culture plates coated with the respective antigen were seeded with up to 6x10⁶ cells per 4 ml medium and allowed to bind for one hour at 37 °C in the incubator. Non-adherent cells were removed by carefully washing the wells 1-2 times with 1x PBS. The remaining sticky cells were detached by trypsin for 10 min. at 37 °C in the incubator and then washed twice in media. The cells were kept on ice until the immune fluorescence staining.

## Claims

1. Method for obtaining a B-cell comprising the following steps:
a) obtaining B-cells from the blood of a rabbit,
b) labeling IgG⁺-B-cells and/or CD138⁺-B-cells,
c) incubating the B-cells at 37 °C for one hour in co-cultivation medium prior to depositing the labeled B-cells as single cells,
d) co-cultivating the single deposited cells with a feeder cell in a co-cultivation medium,
e) selecting a B-cell proliferating in step d) and thereby obtaining a B-cell.

2. Method according to claim 1, **characterized in that** the method comprises the step of centrifuging the single cell deposited cells prior to the co-cultivating.

3. Method according to any one of the preceding claims, **characterized in that** the method comprises prior to the labeling step the following step: ab) panning the B-cells with immobilized antigen.

4. Method according to any one of the preceding claims, **characterized in that** the co-cultivating is in a polystyrene multi well plate with wells coated with a non-fibrous substrate prepared from a blend of polymer plastic resin and amphipathic molecules.

5. Method according to any one of the preceding claims, **characterized in that** obtaining the B-cells is by a density gradient centrifugation.

6. Method according to any one of the preceding claims, **characterized in that** the feeder cell is a murine EL-4 B5 cell.

7. Method according to any one of the preceding claims, **characterized in that** the co-cultivation medium comprises a feeder mix.

8. Method according to claim 7, **characterized in that** said feeder mix is a thymocyte cultivation supernatant.

9. Method according to claim 7, **characterized in that** said feeder mix comprises interleukin-1beta, and tumor necrosis factor alpha and at least one compound selected from interleukin-2, interleukin-10, Staphylococcus aureus strain Cowan cells, interleukin-21, BAFF, interleukin-6, interleukin-4, 5-(4-phenoxybutoxy) psoralene and other stimulating compounds that increase the IgG productivity of the B-cell clone without decreasing the number of IgG⁺ wells.

10. Method for producing an antibody comprising the following steps
a) providing a population of mature B-cells obtained from the blood of a rabbit,
b) labeling IgG⁺-B-cells and/or CD138⁺-B-cells with at least one fluorescence dye,
c) incubating the B-cells at 37 °C for one hour in co-cultivation medium prior to depositing single cells of the labeled population of B-cells in individual containers,
d) cultivating the deposited individual B-cells in the presence of feeder cells and a feeder mix,
e) determining the binding specificity of the antibodies secreted in the cultivation medium of the individual B-cells,
f) determining the amino acid sequence of the variable light and heavy chain domain of specifically binding antibodies by a reverse transcriptase PCR and nucleotide sequencing, and thereby obtaining a monoclonal antibody variable light and heavy chain domain encoding nucleic acid,
g) introducing the monoclonal antibody light and heavy chain variable domain encoding nucleic acid in an expression cassette for the expression of an antibody,
h) introducing the nucleic acid in a cell,
i) cultivating the cell and recovering the antibody from the cell or the cell culture supernatant and thereby producing an antibody.

## Patentansprüche

1. Verfahren zum Erhalten einer B-Zelle, das die folgenden Schritte umfasst:
a) Gewinnen von B-Zellen aus dem Blut eines Kaninchens,
b) Markieren der IgG⁺-B-Zellen und/oder der CD138⁺-B-Zellen,
c) Inkubieren der B-Zellen bei 37°C für eine Stunde in Ko-Züchtungsmedium, bevor die markierten B-Zellen als einzelne Zellen deponiert werden,
d) Ko-Züchten der einzelnen deponierten Zellen mit einer Feeder-Zelle in einem Ko-Züchtungsmedium,
e) Selektieren einer B-Zelle, die sich in Schritt d) vermehrt, wodurch eine B-Zelle erhalten wird.

2. Verfahren nach Anspruch 1, **dadurch gekennzeichnet, dass** das Verfahren vor der Ko-Züchtung den Schritt des Zentrifugierens der einzelnen deponierten Zellen umfasst.

3. Verfahren nach einem der vorangehenden Ansprüche, **dadurch gekennzeichnet, dass** das Verfahren vor dem Markierungsschritt den folgenden Schritt umfasst: ab) Panning der B-Zellen mit immobilisiertem Antigen.

4. Verfahren nach einem der vorangehenden Ansprüche, **dadurch gekennzeichnet, dass** das Ko-Züchten in einer Multiwellplatte aus Polystyrol mit Vertiefungen stattfindet, die mit einem nicht-fibrösen Substrat beschichtet sind, das aus einem Gemisch aus Polymer-Kunstharz und amphipatischen Molekülen hergestellt ist.

5. Verfahren nach einem der vorangehenden Ansprüche, **dadurch gekennzeichnet, dass** die B-Zellen mittels Dichtegradientenzentrifugation erhalten werden.

6. Verfahren nach einem der vorangehenden Ansprüche, **dadurch gekennzeichnet, dass** die Feeder-Zelle eine murine EL-4 B5-Zelle ist.

7. Verfahren nach einem der vorangehenden Ansprüche, **dadurch gekennzeichnet, dass** das Ko-Züchtungsmedium ein Feeder-Gemisch umfasst.

8. Verfahren nach Anspruch 7, **dadurch gekennzeichnet, dass** das Feeder-Gemisch ein Thymocyten-Kulturüberstand ist.

9. Verfahren nach Anspruch 7, **dadurch gekennzeichnet, dass** das Feeder-Gemisch Interleukin-1beta und Tumornekrosefaktor alpha und mindestens eine Verbindung umfasst, ausgewählt aus Interleukin-2, Interleukin-10, Zellen des Staphylococcus aureus-Stamms Cowan, Interleukin-21, BAFF, Interleukin-6, Interleukin-4, 5-(4-Phenoxybutoxy)psoralen und anderen stimulierenden Verbindungen, die die IgG-Produktivität des B-Zell-Clons erhöhen, ohne dass sich die Anzahl der IgG⁺-Vertiefungen vermindert.

10. Verfahren zur Herstellung eines Antikörpers, umfassend die folgenden Schritte
a) Bereitstellen einer Population von reifen B-Zellen, die aus dem Blut eines Kaninchens erhalten wurden,
b) Markieren der IgG⁺-B-Zellen und/oder der CD138⁺-B-Zellen mit mindestens einem Fluoreszenzfarbstoff,
c) Inkubieren der B-Zellen für eine Stunde bei 37°C in einem Ko-Züchtungsmedium, bevor die einzelnen Zellen der markierten B-Zell-Population in individuellen Behältern deponiert werden,
d) Züchten der deponierten einzelnen B-Zellen in Anwesenheit von Feeder-Zellen und eines Feeder-Gemischs,
e) Bestimmen der Bindungsspezifität der Antikörper, die ins Züchtungsmedium der einzelnen B-Zellen sekretiert werden,
f) Bestimmen der Aminosäuresequenz der variablen Domäne der leichten und schweren Kette spezifisch bindender Antikörper mittels einer Reverse-Transkriptase-PCR und Nucleotidsequenzierung, wodurch eine Nucleinsäure erhalten wird, die die variable Domäne der leichten und schweren Kette eines monoclonalen Antikörpers codiert,
g) Einbringen der Nucleinsäure, die die variable Domäne der leichten und schweren Kette eines monoclonalen Antikörpers codiert, in eine Expressionskassette zur Expression eines Antikörpers,
h) Einbringen der Nucleinsäure in eine Zelle,
i) Züchten der Zelle und Gewinnen des Antikörpers aus der Zelle oder dem Zellkulturüberstand, wodurch ein Antikörper hergestellt wird.

## Revendications

1. Procédé d'obtention d'un lymphocyte B, comprenant les étapes suivantes:
a) obtention de lymphocytes B à partir du sang d'un lapin,
b) marquage des lymphocytes B IgG⁺ et/ou des lymphocytes B CD138⁺,
c) incubation des lymphocytes B à 37°C pendant une heure dans un milieu de co-culture avant le dépôt des lymphocytes B marqués sous forme de cellules isolées,
d) co-culture des cellules isolées déposées avec une cellule nourricière dans un milieu de co-culture,
e) sélection d'un lymphocyte B proliférant dans l'étape d) et par suite obtention d'un lymphocyte B.

2. Procédé selon la revendication 1, **caractérisé en ce que** le procédé comprend l'étape de centrifugation des lymphocytes B déposés sous forme de cellules isolées avant la co-culture.

3. Procédé selon l'une quelconque des revendications précédentes, **caractérisé en ce que** le procédé comprend, avant l'étape de marquage, l'étape suivante: ab) adhérence sur plastique des lymphocytes B avec un antigène immobilisé.

4. Procédé selon l'une quelconque des revendications précédentes, **caractérisé en ce que** la co-culture s'effectue dans une plaque multipuits en polystyrène revêtue d'un substrat non fibreux préparé à partir d'un mélange d'une résine plastique polymère et de molécules amphipathiques.

5. Procédé selon l'une quelconque des revendications précédentes, **caractérisé en ce que** l'obtention des lymphocytes B s'effectue par une centrifugation à gradient de densité.

6. Procédé selon l'une quelconque des revendications précédentes, **caractérisé en ce que** la cellule nourricière est une cellule EL-4 B5 de souris.

7. Procédé selon l'une quelconque des revendications précédentes, **caractérisé en ce que** le milieu de co-culture comprend un mélange nourricier.

8. Procédé selon la revendication 7, **caractérisé en ce que** ledit mélange nourricier est un surnageant de culture de thymocytes.

9. Procédé selon la revendication 7, **caractérisé en ce que** ledit mélange nourricier comprend de l'interleukine-1β, et du facteur de nécrose tumorale α et au moins un composé choisi parmi l'interleukine-2, l'interleukine-10, des cellules de souche Cowan de *Staphylococcus aureus,* l'interleukine-21, le BAFF, l'interleukine-6, l'interleukine-4, le 5-(4-phénoxybutoxy)psoralène et d'autres composés stimulants qui augmentent la productivité en IgG du clone de lymphocyte B sans réduire le nombre de puits IgG⁺.

10. Procédé de production d'un anticorps, comprenant les étapes suivantes:
a) fourniture d'une population de lymphocytes B matures obtenus à partir du sang d'un lapin,
b) marquage des lymphocytes B IgG⁺ et/ou des lymphocytes B CD138⁺ avec au moins un colorant fluorescent,
c) incubation des lymphocytes B à 37°C pendant une heure dans un milieu de co-culture avant le dépôt de cellules isolées de la population marquée de lymphocytes B dans des récipients individuels,
d) culture des lymphocytes B individuels déposés en présence de cellules nourricières et d'un mélange nourricier,
e) détermination de la spécificité de liaison des anticorps sécrétés dans le milieu de culture des lymphocytes B individuels,
f) détermination de la séquence d'aminoacides du domaine variable de chaîne légère et lourde d'anticorps à liaison spécifique par une PCR avec transcriptase inverse et séquençage des nucléotides, et par suite obtention d'un acide nucléique codant pour le domaine variable de chaîne lourde et légère d'un anticorps monoclonal,
g) introduction de l'acide nucléique codant pour le domaine variable de chaîne légère et lourde d'un anticorps monoclonal dans une cassette d'expression pour l'expression d'un anticorps,
g) introduction de l'acide nucléique dans une cellule,
i) culture de la cellule et récupération de l'anticorps à partir de la cellule ou du surnageant de culture de la cellule et par suite production d'un anticorps.
